# EUROPEAN PATENT APPLICATION

(11) **EP 4 699 654 A1**
(43) Date of publication of application: **25.02.2026**
(21) Application number: 24792817.9
(22) Date of filing: 23.02.2024
(51) Int. Cl.: A61N 7/02, A61N 7/00

(54) **ULTRASONIC DEVICE CARTRIDGE ENABLING PROCEDURE AT MULTIPLE PROCEDURE DEPTHS, AND CONTROL METHOD THEREFOR**

(30) Priority: 19.04.2023 KR 20230051462; 29.01.2024 KR 20240013294
(71) Applicant: VIOL CO., LTD., Seongnam-si, Gyeonggi-do 13510 (KR)
(72) Inventor: LEE, Eunji, Seongnam-si Gyeonggi-do 13286 (KR); SONG, Yumi, Seongnam-si Gyeonggi-do 13453 (KR); PARK, Yong Seok, Seoul 06326 (KR); LEE, Sangjin, Anyang-si Gyeonggi-do 13510 (KR)
(74) Representative: Maiwald GmbH
(86) International application number: PCT/KR2024/002413
(87) International publication number: WO 2024/219630

(57) **Abstract**

A cartridge for an ultrasound apparatus according to the present invention includes: a cartridge main body; a transducer that is provided in the cartridge main body and generates a high-intensity focused ultrasound; and a moving device that is provided in the cartridge main body and moves the transducer, wherein the moving device includes: a moving main body moving along a first direction; a moving block that is connected to the moving main body and moving together with the transducer by being engaged with the transducer; a first moving member that moves the moving block along the first direction and moves a focus of the high-intensity focused ultrasound along the first direction; and a second moving portion that moves the moving block along a direction inclined with respect to and moves the focus of the high-intensity focused ultrasound along a second direction that is perpendicular to the first direction.

## Description

### [Technical Field]

The present invention relates to a cartridge for an ultrasound apparatus and a control method thereof, and more particularly, it relates to a cartridge for an ultrasound apparatus capable of performing treatment at multiple treatment depths and a control method thereof.

### [Background Art]

Recently, various treatments for skin care and obesity treatment have been developed, and among these various treatments, interest in a treatment performed in a non-invasive manner is increasing.

An ultrasound is widely used for non-invasive treatments, and among them, an ultrasound apparatus using a high intensity focused ultrasound (HIFU) is recently attracting attention. For example, an ultrasound apparatus that non-invasively performs a skin beauty treatment such as face lifting or skin tightening by irradiating high-intensity focused ultrasound into the skin tissue, or an ultrasound apparatus that non-invasively performs obesity treatment by irradiating high-intensity focused ultrasound into the subcutaneous fat layer to non-invasively burn or melt and decompose fatty tissue is being developed.

The HIFU apparatus used for a skin care treatment includes a hand piece and an ultrasound control system, and the hand piece contains a cartridge containing a transducer that generates ultrasounds.

Since the transducer that generates the ultrasound in the cartridge for the ultrasound apparatus is fixed to a treatment part of one treatment depth and performs a straight line reciprocal motion, the cartridge needs to be replaced to perform the treatment in a treatment part of a different treatment depth. In this way, when replacing the cartridge, the procedure becomes complicated and the procedure time becomes longer, causing inconvenience for both the operator and the recipient.

### [Disclosure]

### [Technical Problem]

The present invention aims to provide a cartridge for an ultrasound apparatus that can simplify a treatment and shorten a treatment time.

In addition, the present invention aims to provide a control method for a cartridge for an ultrasound apparatus that can easily control a treatment area to maximize treatment efficiency.

### [Technical Solution]

A cartridge for an ultrasound apparatus according to the present invention includes: a cartridge main body; a transducer that is provided in the cartridge main body and generates a high-intensity focused ultrasound; and a moving device that is provided in the cartridge main body and moves the transducer, wherein the moving device includes: a moving main body moving along a first direction; a moving block that is connected to the moving main body and moving together with the transducer by being engaged with the transducer; a first moving member that moves the moving block along the first direction and moves a focus of the high-intensity focused ultrasound along the first direction; and a second moving portion that moves the moving block along a direction inclined with respect to and moves the focus of the high-intensity focused ultrasound along a second direction that is perpendicular to the first direction.

The first moving member may include: a moving connection portion that connects a driver of a hand piece main body providing driving torque and the moving block; and a pair of guide rods that are connected with the moving connection portion and guide the moving block to move along the first direction

The moving connection portion may include: a connection main body that reciprocally moves along the first direction; a first connection portion that extends from an upper end of the connection main body and is connected with the driver; a second connection portion that is connected with the pair of guide rods at a central portion of the connection main body; and a third connection portion that is engaged with the moving block at a lower end of the connection main body.

The second moving portion may include: a moving pin that connects the moving main body and the moving block and is movable; a fixing pin that is installed in the moving main body in parallel at a distance from the moving pin; and a link that connects the moving pin and the fixing pin to each other, and the moving pin is reciprocally movable along an inclined hole formed along the inclined direction on a side wall of the moving main body.

One end of the link may include a moving hole to which the moving pin is coupled, the other end of the link may include a rotation hole to which the fixing pin is coupled, and a size of the moving hole may be larger than a diameter of the moving pin.

The moving device may further include a stationary member that is provided in an inner wall of the cartridge main body and stops the movement of the moving main body to the first direction, and the stationary member may include a first stationary member and a second stationary member that are opposite to each other in the first direction.

The moving main body may include: a frame including the inclined hole; and a first protruding portion extending in the first direction from the frame and is capable of being in contact with the stationary member.

The moving main body may further include a second protruding portion extending in the second direction from an upper end of the frame and to which the pair of guide rods are inserted, the second protruding portion may include a second protruding portion on one side and a second protruding portion on the other side positioned spaced apart from each other by a distance, and a length of the second connection portion may be shorter than the distance.

The second moving portion may include a moving pin that connects the moving main body and the moving block and is movable, and the moving pin may be reciprocally movable along a two-stage guide hole formed on a side wall of the moving main body.

The moving main body may include: a frame including the two-stage guide hole; and a second protruding portion extending in the second direction from an upper end of the frame and to which the pair of guide rods are inserted.

The second protruding portion may include a second protruding portion on one side and a second protruding portion on the other side positioned spaced apart from each other by a distance, and a length of the second connection portion may be shorter than the distance.

The two-stage guide hole may include a first horizontal guide hole and a second horizontal guide hole that are disposed spaced apart from each other along the first direction and are formed at different heights along the second direction, and an inclined connection hole formed along the slope direction and connecting the first horizontal guide hole and the second horizontal guide hole.

In addition, a cartridge for an ultrasound apparatus according to another embodiment includes: a cartridge main body; a transducer that is provided in the cartridge main body and generates a high-intensity focused ultrasound; and a moving device that is provided in the cartridge main body and moves the transducer, wherein the moving device may include: a fixed main body that is fixed within the cartridge main body and includes a moving guide groove on the inside; a moving block that is connected to the moving main body and moving together with the transducer by being engaged with the transducer; a first moving member that moves the moving block along the first direction and moves a focus of the high-intensity focused ultrasound along the first direction; and a second moving portion that moves the moving block along a direction inclined with respect to and moves the focus of the high-intensity focused ultrasound along a second direction that is perpendicular to the first direction.

The first moving member may include: a moving connection portion that connects a driver of a hand piece main body providing driving torque and the moving block; and a pair of guide rods that are provided in the fixed main body and connected to the moving connection portion to guide the moving block to move along the first direction.

The moving connection portion may include: a connection main body that reciprocally moves along the first direction; a first connection portion that extends from an upper end of the connection main body and is connected with the driver; a second connection portion that is connected with the pair of guide rods at a central portion of the connection main body; and a third connection portion that is engaged with the moving block at a lower end of the connection main body.

The moving guide groove may include: a first horizontal guide groove and a second horizontal guide groove that are formed at different heights and face each other; and a first inclined guide groove and a second inclined guide groove that are formed along an inclination direction, facing each other and connecting the first horizontal guide groove and the second horizontal guide groove.

The second moving portion may include: a moving link that is connected with the third connection portion and moves along the movement guide groove by being inserted into the moving guide groove; a connection pin that connects the moving link and the third connection portion; and a middle pin connecting the moving link and the moving block.

In addition, a control method of a cartridge for an ultrasound apparatus that adjusts a treatment depth of high-intensity focused ultrasound generated from a transducer and irradiated to a treatment target, includes: setting a first treatment length, which is a distance between a first treatment start point where the high-intensity focused ultrasound is irradiated at a first treatment depth and a first treatment end point, and a second treatment length, which is a distance between a second treatment start point where the high-intensity focused ultrasound is irradiated at a second treatment depth that is different from the first treatment depth and a second treatment end point; moving the transducer from a first movement start point to a first movement end point and irradiating the treatment target with the high-intensity focused ultrasound for the first treatment length; changing the first treatment depth to the second treatment depth by changing a focal distance of the transducer; moving the transducer to a second movement start point; and irradiating the treatment target with the high-intensity focused ultrasound at the second treatment length while moving the transducer from the second movement start point to a second movement end point, wherein the first treatment end point corresponding to the first movement end point may be changeable by adjusting the first movement end point of the transducer.

The first movement start point may correspond to the first treatment start point, the second movement start point may correspond to the second treatment start point, the second movement end point may correspond to the second treatment end point, and the second treatment start point may be changeable by adjusting the second movement start of the transducer.

The control method of the cartridge for the ultrasound apparatus may further include: moving the transducer from the first movement end point to a maximum treatment possible end point; and moving the transducer to a first focal distance change point from the maximum treatment possible end point, wherein the maximum treatment possible end point may be a maximum treatment possible point to which the transducer performs treatment while moving.

The control method of the cartridge for the ultrasound apparatus may further include: moving the transducer to a second focal distance change point from the second movement end point; changing the second treatment depth to the first treatment depth by changing the focal distance of the transducer; and moving the transducer to the first movement start point.

The second movement end point may be the same as the first movement start point.

The first treatment end point and the second treatment start point may be set to be different from each other.

The first treatment end point and the second treatment start point may be set to be the same.

The first treatment end point and the second treatment start point may be set to be the same as the first movement end point.

The first treatment end point and the second treatment start point may be set to be the same as the maximum treatment possible end point.

A moving device that moves the transducer may include: a moving block that is engaged with the transducer and moves together with the transducer; and a first moving member that moves a focus of the high-intensity focused ultrasound along a first direction while moving the moving block along the first direction, wherein the first moving member reciprocally moves the transducer between the first focal distance change point and the second focal distance change point.

The moving device may further include a second moving point that moves a focus of the high-intensity focused ultrasound in a second direction that is perpendicular to the first direction while moving the moving block along an inclined direction that is inclined with respect to the first direction, and the second moving portion may change a focal distance of the transducer.

### [Advantageous Effects]

According to the embodiment, the cartridge for the ultrasound apparatus can treat a plurality of treatment depths with a single cartridge, and thus a treatment procedure is simple and a treatment time can be shortened.

In addition, the control method of the cartridge for the ultrasound apparatus according to the embodiment can easily control the treatment area by changing the first treatment end point, which is the end point where high-intensity focused ultrasound is irradiated at the first treatment depth, and the second treatment start point, which is the start point where high-intensity focused ultrasound is irradiated at the second treatment depth which is different from the first treatment depth.

Therefore, since a single cartridge can be used to treat a variety of treatment areas with a plurality of treatment depths, the treatment procedure is simple and treatment efficiency can be maximized.

### [Description of the Drawings]

FIG. 1 is provided for description of a state in which a cartridge for an ultrasound apparatus according to an embodiment is assembled to an ultrasound apparatus.
FIG. 2 is a front view of the cartridge for the ultrasound apparatus according to an embodiment.
FIG. 3 is a perspective view of a transducer and a moving device of FIG. 2.
FIG. 4 is a top plan view of the moving device of FIG. 2.
FIG. 5 is a perspective view of a state in which a moving main body is removed in FIG. 3.
FIG. 6 to FIG. 9 are front views sequentially illustrating a method for changing a treatment depth by moving a transducer of a cartridge for an ultrasound apparatus by a moving device according to an embodiment.
FIG. 10 is a perspective view of a transducer and a moving device of a cartridge for an ultrasound apparatus according to another embodiment.
FIG. 11 is a perspective view of a state in which a moving main body and a moving connection portion in FIG. 10 are removed.
FIG. 12 is provided for description of a moving pin that moves along a two-stage guide hole of the moving main body.
FIG. 13 is a perspective view of a transducer and a moving device of a cartridge for an ultrasound apparatus according to still another embodiment.
FIG. 14 is a perspective view of a fixed main body of FIG. 13.
FIG. 15 is a perspective view of a state in which the fixed main body in FIG. 13 is removed.
FIG. 16 is a schematic flowchart of a control method of a cartridge for an ultrasound apparatus according to an embodiment.
FIG. 17 is provided for description of an embodiment of the control method of the cartridge for the ultrasound apparatus of FIG. 16.
FIG. 18 is provided for description of another embodiment of the control method of the cartridge for the ultrasound apparatus of FIG. 16.
FIG. 19 is provided for description of still another embodiment of the cartridge for the ultrasound apparatus of FIG. 16.
FIG. 20 is provided for description of still another embodiment of the cartridge for the ultrasound apparatus of FIG. 16.

### [Mode for Invention]

the present invention will be described more fully hereinafter with reference to the accompanying drawings, in which embodiments of the invention are shown. As those skilled in the art would realize, the described embodiments may be modified in various different ways, all without departing from the spirit or scope of the present invention.

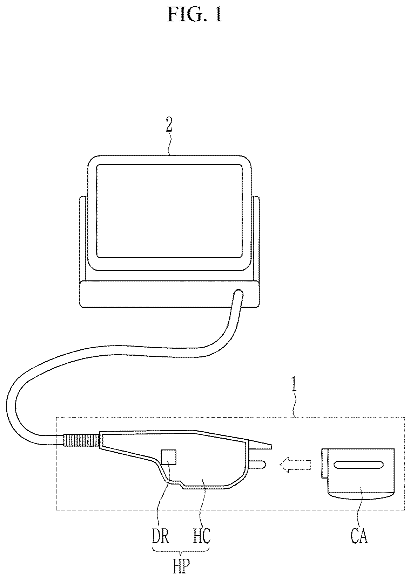

FIG. 1 is provided for description of a state in which a cartridge for an ultrasound apparatus according to an embodiment is assembled to an ultrasound apparatus.

As shown in FIG. 1, an ultrasound apparatus including an ultrasound apparatus cartridge according to an embodiment includes a hand piece 1 and an ultrasound control system 2.

The hand piece 1 may generate a high intensity focused ultrasound (HIFU), and may transmit acoustic energy by focusing the HIFU on a specific treatment part of a subject of a procedure.

The hand piece 1 may include a hand piece main body HP and a cartridge CA. The hand piece main body HP may be connected to the ultrasound control system 2, and the cartridge CA may be coupled to the hand piece main body HP.

The hand piece main body HP may include a hand piece case HC and a driver DR.

The cartridge CA may be coupled to an internal space of the hand piece case HC.

The driver DR is installed inside the hand piece case HC, and may adjust a treatment range of the HIFU irradiated to the treatment subject by moving the position of the cartridge CA coupled to the hand piece case HC. Such a driver DR may include a drive motor and the like.

The cartridge CA may generate the HIFU and irradiate the generated HIFU to a plurality of treatment depths of a treatment part of the treatment subject.

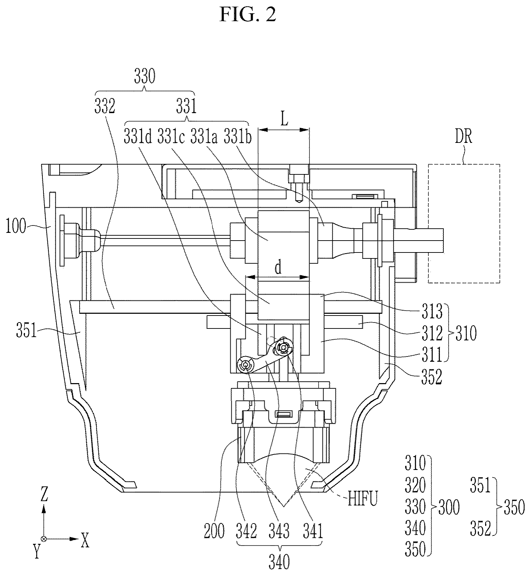

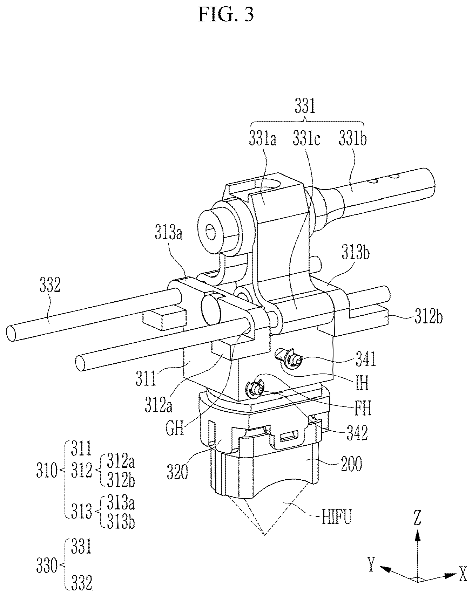

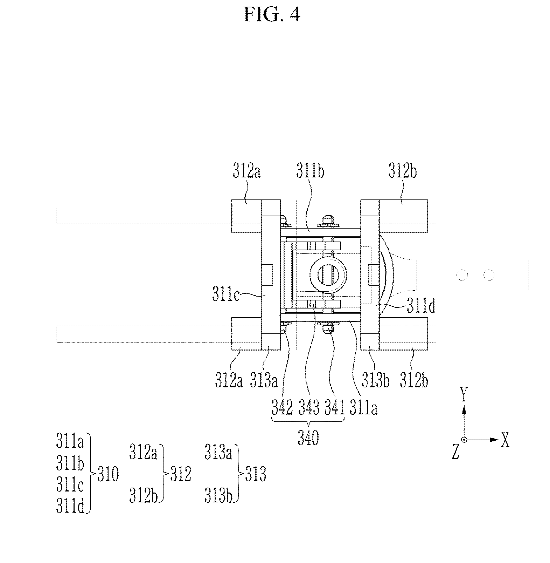

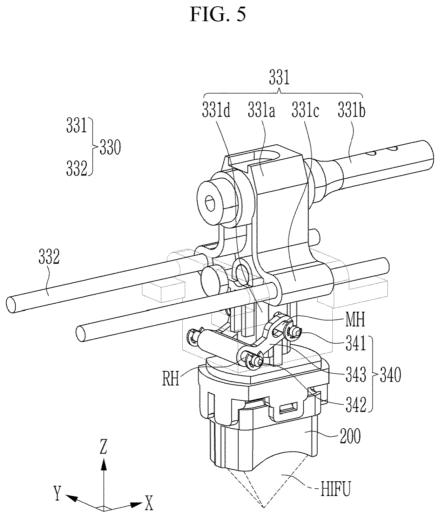

FIG. 2 is a front view of the cartridge for the ultrasound apparatus according to an embodiment. FIG. 3 is a perspective view of the transducer and a moving device of FIG. 2. FIG. 4 is a top plan view of the moving device of FIG. 2. FIG. 5 is a perspective view of a state in which a moving main body is removed in FIG. 3.

As shown in FIG. 2 to FIG. 5, the cartridge for the ultrasound apparatus according to an embodiment may include a cartridge main body 100, a transducer 200, and a moving device 300.

The cartridge main body 100 may have a roughly rectangular hexahedral shape, but is not limited thereto, and may have various shapes. The cartridge main body 100 may have an internal space, in which the transducer 200 and the moving device 300 are installed.

The transducer 200 generates a high-intensity focused ultrasound (HIFU) and may irradiate the generated HIFU to a treatment part of the treatment subject. Since a transducer element (not shown) installed inside the transducer 200 has a concave shape in a second direction Z, the focus of the HIFU may be formed on the axis in the second direction Z.

The moving device 300 may include a moving main body 310, a moving block 320, a first moving member 330, a second moving member 340, and a stationary member 350.

The moving main body 310 may move the moving block 320 and the transducer 200 connected to the moving main body 310 in a first direction X, which is a horizontal direction, while moving along the first direction X.

The moving main body 310 may include a frame 311, a first protruding portion 312, and a second protruding portion 313.

The frame 311 may have a rectangular frame shape with an opening in the second direction Z. The frame 311 may include a first side wall 311a and a second side wall 311a that extend in parallel along the first direction X and face each other, and a third side wall 311c and a fourth side wall 311d that extend in parallel along a third direction Y that is perpendicular to the first direction X and face each other. The first side wall 311a, the second side wall 311b, the third side wall 311c, and the fourth side wall 311d are connected with each other while neighboring each other, and may form a quadrangular frame shape. Here, the first side wall 311a and the second side wall 311b may have inclined holes **IH** inclined with respect to the first direction X.

The first protruding portion 312 may extend in the first direction X from an upper end of the frame 311. Such a first protruding portion 312 may contact the stationary member 350 as the moving main body 310 moves along the first direction X. The first protruding portion 312 may include a first protruding portion 312a on one side and a first protruding portion 312b on the other side.

The second protruding portion 313 may extend from an upper end of the frame 311 in the second direction Z that is simultaneously perpendicular to the first direction X and the third direction Y. Such a second protruding portion 313 may have a penetration hole GH. The second protruding portion 313 may include a second protruding portion 313a on one side and a second protruding portion 313b on the other side, which are disposed spaced apart from each other by a distance d.

The moving block 320 is connected to the moving main body 310 and engaged with the transducer 200, thereby moving together with the transducer 200. That is, an upper end portion of the moving block 320 may be connected to the moving main body 310, and a lower end portion of the moving block 320 may be engaged with the transducer 200.

The first moving member 330 moves the moving main body 310 along the first direction X such that the moving block 320 connected to the moving main body 310 can move along the first direction X. Therefore, the focus of the HIFU generated from the transducer 200 engaged in the moving block 320 may be moved along the first direction X.

The first moving member 330 may include a moving connection portions 331 and a pair of guide rods 332.

The moving connection portion 331 may connect a driver DR of the hand piece main body HP, which provides a driving torque, with the moving block 320. The moving connection portion 331 may include a connection main body 331a that reciprocally moves along the first direction X, a first connection portion 331b that extends along the first direction X from an upper end portion of the connection main body 331a and connected with the driver DR, a second connection portion 331c that is connected with the pair of guide rods 332 at a central portion of the connection main body 331a, and a third connection portion 331d that is engaged with the moving block 320 at a lower end portion of the connection main body 331a.

A length L of the second connection portion 331c may be shorter than a distance d between the second protruding portion 313a on one side and the second protruding portion 313b on the other side. Therefore, the second connection portion 331c may reciprocally move between the second protruding portion 313a on one side and the second protruding portion 313b on the other side.

The pair of guide rods 332 is inserted into the second connection portion 331c of the moving connection portion 331 and the penetration hole GH of the second protruding portion 313 of the moving main body 310 to guide the moving block 320 to move along the first direction X.

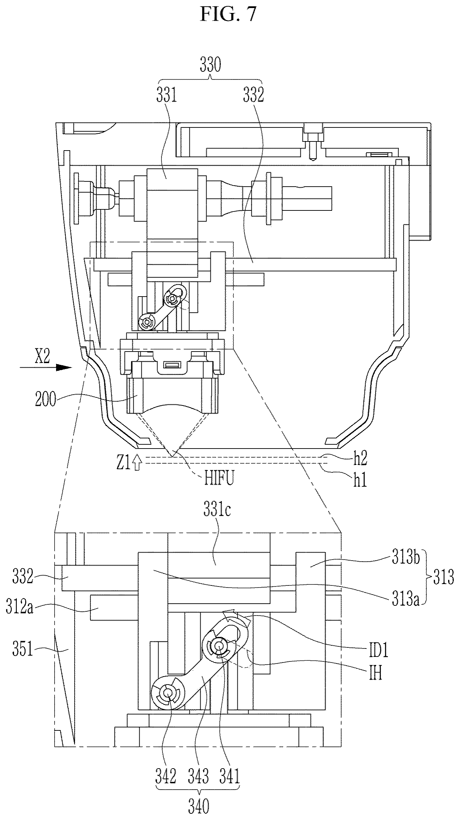

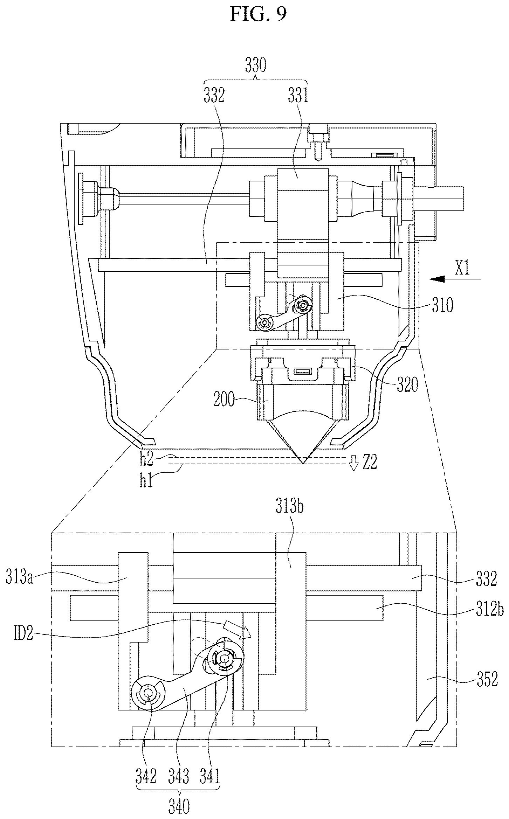

The second moving portion 340 may move the moving block 320 along an inclined direction (refer to ID1 and ID2 in FIG. 7 and FIG. 9) that is inclined with respect to the first direction X. Therefore, eventually, the focus of the HIFU may be moved in the second direction Z.

The second moving portion 340 may include a moving pin 341, a fixing pin 342, and a link 343.

The moving pin 341 may extend in the third direction Y, and may connect the moving main body 310 and the moving block 320. That is, the moving pin 341 may penetrate an inclined hole HI formed along the inclined directions ID1 and ID2 in the first side wall 311a and the second side wall 311b of the moving main body 310, thereby being installed in the moving main body 310. In addition, the moving pin 341 may be engaged with the upper end portion of the moving block 320, while being engaged with the link 343 at the same time. In addition, the moving pin 341 may reciprocally move along the inclined hole IH.

The fixing pin 342 may be installed in the moving main body 310 by penetrating a fixing hole FH formed in the first side wall 311a and the second side wall 311b of the moving main body 310 in a parallel manner and spaced apart from the moving pin 341.

The link 343 may connect the moving pin 341 and the fixing pin 342.

One end of the link 343 may have a moving hole MH to which the moving pin 341 is coupled. The moving hole MH may be greater than a diameter of the moving pin 341 in size. Thus, the moving pin 341 may move along the inclined directions ID1 and ID2 while reciprocally moving inside the moving hole MH.

The other end of the link 343 may have a rotation hole RH to which the fixing pin 342 is coupled.

As described, as the moving pin 341 moves along the inclined hole IH, the moving block 320 engaged with the moving block 320 may move in the inclined directions direction ID1 and ID2. Therefore, the position of the transducer 200 engaged at the lower end of the moving block 320 in the second direction Z can be changed. Since, the position of the focus of the HIFU generated from the transducer 200 in the second direction Z can be changed, a treatment depth, which is the position at which the focus of HIFU is focused on the treatment part of the subject, can be changed. For example, using the moving device 300, the focus of the HIFU may circulate between a treatment depth of 4.5 mm and a treatment depth of 3.0 mm, or between a treatment depth of 6.0 mm and a treatment depth of 4.5 mm.

The stationary member 350 may be installed on an interior wall of the cartridge main body 100 to stop the movement of the moving main body 310 in the first direction X.

The stationary member 350 may include a first stationary member 351 and a second stationary member 352 that are opposite to each other in the first direction X.

The moving main body 310 moving to the left along the first direction X may stop as the first protruding portion 312a on one side of the moving main body 310 contacts the first stationary member 351. In addition, the moving main body 310 moving to the right along the first direction X may stop as the first protruding portion 312b on the other side of the moving main body 310 contacts the second stationary member 352.

The ultrasound control system 2 may control the HIFU generated from the transducer 200.

In this way, the cartridge for the ultrasound apparatus according to an embodiment may perform a plurality of treatment depths with one cartridge, and thus the treatment procedure is simple and the treatment time may be shortened.

Hereinafter, a method for controlling a treatment depth using a cartridge for an ultrasound apparatus according to an embodiment will be described in detail with reference to the accompanying drawings.

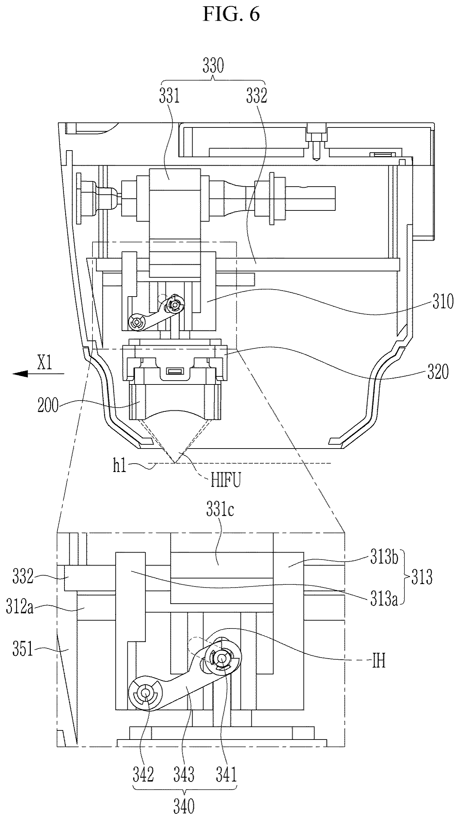

FIG. 6 to FIG. 9 are front views sequentially illustrating a method for changing a treatment depth by moving a transducer of a cartridge for an ultrasound apparatus by a moving device according to an embodiment.

First, as shown in FIG. 6, when the moving connection portion 331 of the first moving member 330 moves to the left in a first direction X1 along the pair of guide rods 332 by the driving torque of the driver DR while maintaining the treatment depth of the HIFU generated from transducer 200 at h1, the moving main body 310 and the moving block 320 connected to the connection portion 331 also move to the left in the first direction X1. In this case, the second connection portion 331c of the moving connection portion 331 moves while maintaining the contact state with the second protruding portion 313b on the other side of the moving main body 310. When the first protruding portion 312a on one side of the moving main body 310 comes into contact with the first stationary member 351 while the moving main body 310 continues to move to the left in the first direction X, the movement of the moving main body 310 stops.

Next, as shown in FIG. 7, the driving torque of the driver DR is continuously applied to the moving main body 310, but the moving main body 310 stops by the first stationary member 351, and therefore, the second connection portion 331c of the moving connection portion 331 is separated from the other-side second protruding portion 313b of the moving main body 310 and moves to the left along the first direction X until it contacts the second protruding portion 313a on one side. As the second connection portion 331c moves, the moving block 320 engaged in the third connection portion 331d of the moving connection portion 331 also moves. Here, the second connection portion 331c moves to the left in the first direction X along the pair of guide rods 332, but the moving block 320 is connected with the moving pin 341 and the link 343 and therefore the moving block 320 is guided by the upside inclined hole IH of the frame 311 of the moving main body 310 and moves to an upward second direction Z1 along the inclined direction ID1. Accordingly, the position of the transducer 200 connected to the moving block 320 in the second direction Z moves upward, and thus the treatment depth of the HIFU generated from the transducer 200 changes to h2.

In addition, when the moving connection portion 331 of the first moving member moves to a rightward by the driving torque of the driver DR while the treatment depth of the HIFU is maintain at h2, the moving main body 310 and the moving block 320 connected to the moving connection portion 331 also move to the rightward first direction X2. In this case, while the second connection portion 331c of the moving connection portion 331 is in contact with the second protruding portion 313a on one side of the moving main body 310, the first protruding portion 312a on one side of the moving main body 310 may be separated from the first stationary member 351.

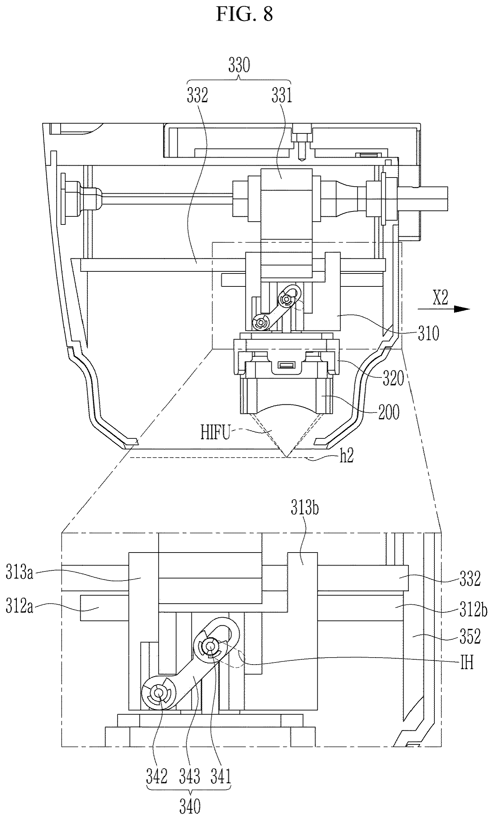

Next, as shown in FIG. 8, when the moving main body 310 continuously moves to the rightward first direction X2 and the first protruding portion 312b on the other side of the moving main body 310 contacts the second stationary member 352 while the treatment depth of the HIFU maintains at h2, the movement of the moving main body 310 stops.

Next, as shown in FIG. 9, although the driving torque of the driver DR is continuously applied to the moving main body 310, the movement of the moving main body 310 is stopped by the second stationary member 352, and therefore, the second connection portion 331c of the moving connection portion 331 is separated from the second protruding portion 313a on one side of the moving main body 310 by the driving torque of the driver DR and moves to the second protruding portion 313b on the other side to contact the second protruding portion 313b on the other side. As the second connection portion 331c moves, the moving block 320 connected to the third connection portion 331d of the moving connection portion 331 also moves. Here, the second connection portion 331c moves to the rightward first direction X along the pair of guide rods 332, but the moving block 320 is connected with the moving pin 341 and the link 343, and therefore the moving block 320 is guided by the inclined hole IH of the frame 311 of the moving main body 310 and moves to a downward second direction Z2 along the downside inclined direction ID2. Accordingly, since the position of the transducer 200 connected to the moving block 320 in the second direction Z moves downward, the treatment depth of the HIFU generated from the transducer 200 may be changed to h1.

Meanwhile, in the embodiment, the moving pin of the second moving portion is capable of reciprocal movement along the inclined hole, but another embodiment in which the moving pin of the second moving portion is capable of reciprocal movement along the two-stage guide hole is also possible.

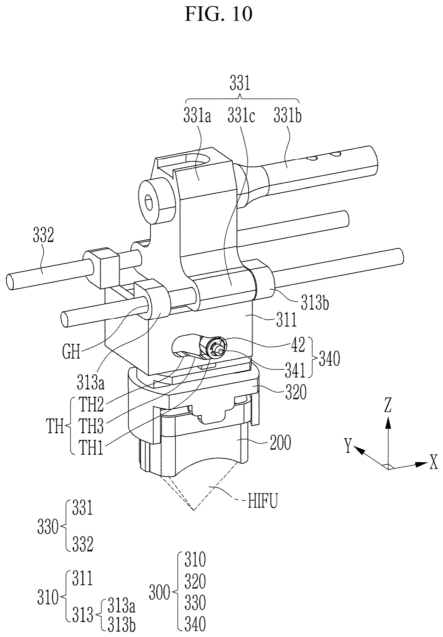

Hereinafter, referring to FIG. 10 to FIG. 12, a cartridge for an ultrasound apparatus according to another embodiment of the present invention will be described in detail.

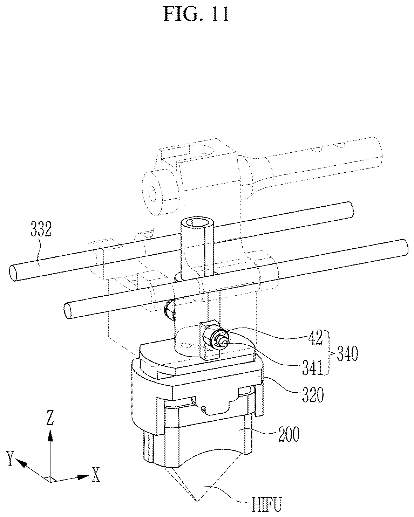

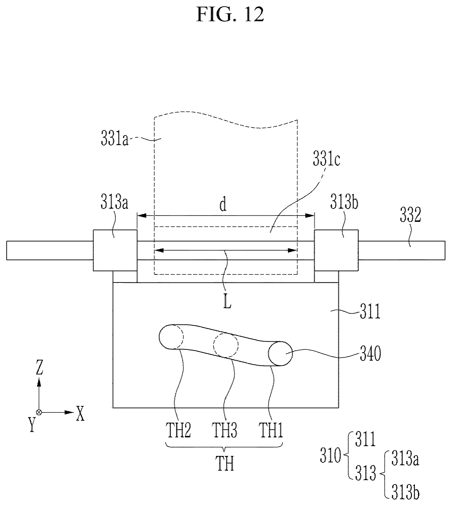

FIG. 10 is a perspective view of a transducer and a moving device of a cartridge for an ultrasound apparatus according to another embodiment, FIG. 11 is a perspective view of a state in which a moving main body and a moving connection portion in FIG. 10 are removed, and FIG. 12 is provided for description of a moving pin that moves along a two-stage guide hole of the moving main body.

The embodiment shown in FIG. 10 to FIG. 12 is substantially the same as the embodiment shown in FIG. 2 to FIG. 5, except for a structure of a moving device, and therefore the repeated description will be omitted.

As shown in FIG. 2, FIG. 10, and FIG. 11, a cartridge for an ultrasound apparatus according to another embodiment may include a cartridge main body 100, a transducer 200, and a moving device 300.

The moving device 300 may include a moving main body 310, a moving block 320, a first moving member 330, and a second moving portion 340.

The moving main body 310 may move the moving block 320 and the transducer 200 connected to the moving main body 310 along a first direction X, which is a horizontal direction, while moving along the first direction X.

The moving main body 310 may include a frame 311 and a second protruding portion 313.

A side wall of frame 311 may have a two-stage guide hole TH. The two-stage guide hole TH may include a first horizontal guide hole TH1, a second horizontal guide hole TH2, and an inclined connection hole TH3.

The first horizontal guide hole TH1 and the second horizontal guide hole TH2 may be disposed apart from each other along the first direction X. In addition, the first horizontal guide hole TH1 and the second horizontal guide hole TH2 may be formed at different heights along a second direction Z.

The inclined connection hole TH3 connects the first horizontal guide hole TH1 and the second horizontal guide hole TH2 to each other and may be formed along an inclined direction inclined with respect to the first direction X.

The second protruding portion 313 may extend in the second direction Z from an upper end of a frame 311. Such a second protruding portion 313 may have a penetration hole GH. The second protruding portion 313 may include a second protruding portion 313a on one side and a second protruding portion 313b on the other side, which are disposed apart from each other by a distance d.

The moving block 320 is connected to the moving main body 310 and engages with the transducer 200 such that it can move together with the transducer 200. That is, an upper end of the moving block 320 may be connected to the moving main body 310, and a lower end of the moving block 320 may be engaged with the transducer 200.

The first moving member 330 may move the moving block 320 connected to the moving main body 310 along the first direction X by moving the moving main body 310 along the first direction X. Accordingly, the focus of high intensity focused ultrasound (HIFU) generated from the transducer 200 engaged in the moving block 320 may be moved along the first direction X.

The first moving member 330 may include a moving connection portion 331 and a pair of guide rods 332.

The moving connection portion 331 may connect a driver DR of a hand piece main body HP, which provides driving torque, and the moving block 320. The moving connection portion 331 may include a connection main body 331a that reciprocally moves along the first direction X, a first connection portion 331b that extends in the first direction X from an upper end of the connection main body 331a and is connected to the driver DR, a second connection portion 331c connected with a pair of guide rods 332 at a central portion of the connection main body 331a, and a third connection portion 331d (refer to FIG. 5) engaged with the moving block 320 at a lower end of the connection main body 331a.

A length L of the second connection portion 331c may be shorter than the distance d between the second protruding portion 313a on one side and the second protruding portion 313b on the other side. Accordingly, the second connection portion 331c may reciprocally move between the second protruding portion 313a on one side and the second protruding portion 313b on the other side.

The pair of guide rods 332 may be inserted into the second connection portion 331c of the moving connection portion 331 and the penetration hole GH of the second protruding portion 313 of the moving main body 310 to guide the moving block 320 to move along the first direction X.

The second moving portion 340 may include a moving pin 341 and a pin fixing portion 42.

The moving pin 341 extends in a third direction Y and may connect the moving main body 310 and the moving block 320. That is, the moving pin 341 may be installed in the moving main body 310 by penetrating the two-stage guide hole TH formed in the side wall of the moving main body 310. In addition, the moving pin 341 may be engaged with an upper end of the moving block 320. The moving pin 341 may reciprocally move along the two-stage guide hole TH.

As shown in FIG. 12, when the moving pin 341 is positioned in the first horizontal guide hole TH1, a treatment depth of the high-intensity focused ultrasound (HIFU) generated from the transducer 200 engaged in the moving block 320 connected to the moving pin 341 may be h1. In addition, when the moving pin 341 moves in an inclined direction along the inclined connection hole TH3 and is positioned in the second horizontal guide hole TH2, the treatment depth of the HIFU generated from the transducer 200 engaged in the moving block 320 connected to the moving pin 341 may be h2.

In this way, as the moving pin 341 moves along the two-stage guide hole TH, the treatment depth of the HIFU generated from the transducer 200 engaged in the moving block 320 connected to the moving pin 341 can be changed.

Meanwhile, in the embodiment, the moving device moves along the first direction and includes the moving main body having the inclined hole, but another embodiment in which a fixed main body fixed within the cartridge main body and includes a moving guide groove thereinside is also possible.

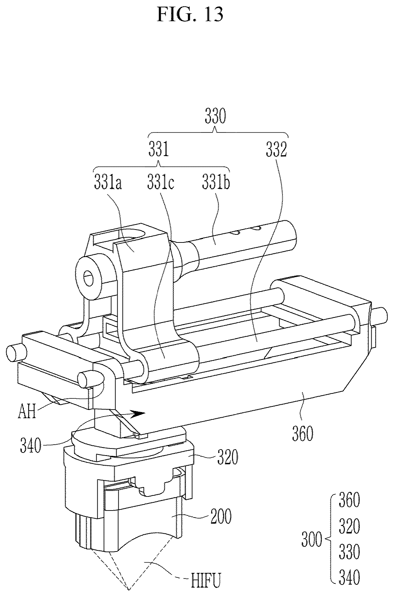

Hereinafter, referring to FIG. 13 to FIG. 15, a cartridge for an ultrasound apparatus according to still another embodiment of the present invention will be described in detail.

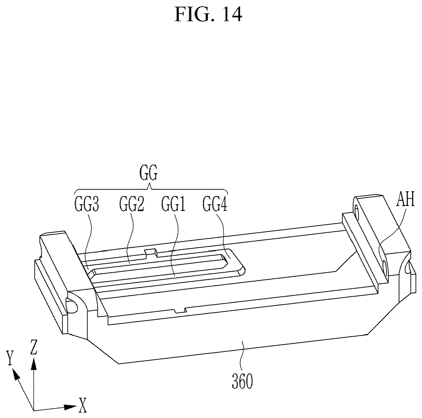

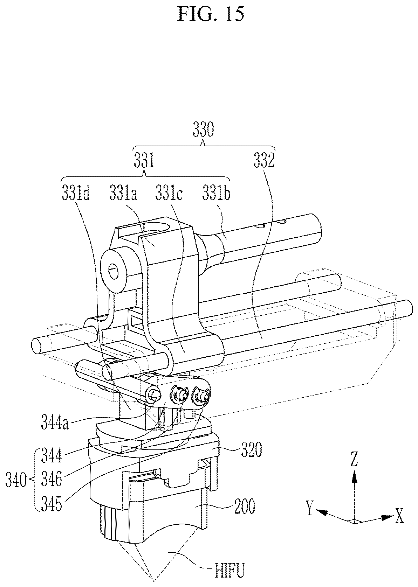

FIG. 13 is a perspective view of a transducer and a moving device of a cartridge for an ultrasound apparatus according to still another embodiment, FIG. 14 is a perspective view of a fixed main body of FIG. 13, and FIG. 15 is a perspective view of a state in which the fixed main body in FIG. 13 is removed.

The embodiment shown in FIG. 13 to FIG. 15 is substantially the same as the embodiment shown in FIG. 2 to FIG. 5, except for a structure of a moving device, and therefore the repeated description will be omitted.

As shown in FIG. 2 and FIG. 13 to FIG. 15, a cartridge for an ultrasound apparatus according to still another embodiment may include a cartridge main body 100, a transducer 200, and a moving device 300.

The moving device 300 may include a fixed main body 360, a moving block 320, a first moving member 330, and a second moving portion 340.

The fixed main body 360 is fixed within the cartridge main body 100 and may have a moving guide groove GG on the inside.

The moving guide groove GG may include a first horizontal guide groove GG1, a second horizontal guide groove GG2, a first inclined guide groove GG3, and a second inclined guide groove GG4.

The first horizontal guide groove GG1 and the second horizontal guide groove GG2 are opposite to each other and may be formed at different heights with a second direction Z as a reference.

The first inclined guide groove GG3 and the second inclined guide groove GG4 are opposite to each other and may connect the first horizontal guide groove GG1 and the second horizontal guide groove GG2 to each other. The first inclined guide groove GG3 and the second inclined guide groove GG4 may be formed along an inclined direction inclined with respect to the first direction X.

The moving block 320 is connected to the fixed main body 360 and is engaged with the transducer 200, thereby moving together with the transducer 200. That is, an upper end of the moving block 320 may be connected to the fixed main body 360, and a lower end of the moving block 320 may be engaged with the transducer 200.

The first moving member 330 may include a moving connection portion 331 and a pair of guide rods 332.
moving connection portion 331 may include a connection main body 331a reciprocally moving along a first direction X, first connection portion 331b extending in the first direction X from an upper end of the connection main body 331a and is connected to a driver DR, a second connection portion 331c connected with pair of guide rods 332 at a central portion of the connection main body 331a, and engaged with the moving block 320 at a lower end of the connection main body 331a.

The pair of guide rods 332 may be installed on the fixed main body 360. The pair of guide rods 332 may be inserted into the second connection portion 331c of the moving connection portion 331 and an insertion hole AH of the fixed main body 360 to guide the moving block 320 to move along the first direction X.

The second moving portion 340 may include a moving link 344, a connection pin 345, and a middle pin 346.

The moving link 344 may be connected with the third connection portion 331d of the moving connection portion 331. One end of the moving link 344 may have a link pin 344a. The link pin 344a may be inserted into the moving guide groove GG of the fixed main body 360 and move along the moving guide groove GG. Accordingly, the moving connection portion 331 connected to the moving link 344 and the moving block 320 engaged in the moving connection portion 331 may also move together according to the movement of the moving link 344.

The connection pin 345 may connect the other end of the moving link 344 and the third connection portion 331d.

The middle pin 346 may connect a middle part of the moving link 344 and the moving block 320. When the link pin 344a of the moving link 344 moves along the first inclined guide groove GG3 and the second inclined guide groove GG4, the moving block 320, engaged with the moving link 344 via the middle pin 346, may change position in a second direction Z.

For example, when the link pin 344a of the moving link 344 is disposed in the first horizontal guide groove GG1, a treatment depth of the high-intensity focused ultrasound (HIFU) generated from the transducer 200 engaged in the moving block 320 connected to the moving link 344 may be h1. In addition, when the link pin 344a of the moving link 344 moves in an inclined direction along the first inclined guide groove GG3 or the second inclined guide groove GG4 and is positioned in the second horizontal guide groove GG2, the treatment depth of the HIFU generated from the transducer 200 engaged in the moving block 320 connected to the moving link 344 may be h2.

As described, as the link pin 344a of the moving link 344 moves along the moving guide groove GG, the treatment depth of the HIFU generated from the transducer 200 engaged in the moving block 320 connected to the moving link 344 may be changed.

Hereinafter, a method of controlling an ultrasound apparatus using the ultrasound control system 2 will be described in detail with reference to the drawing.

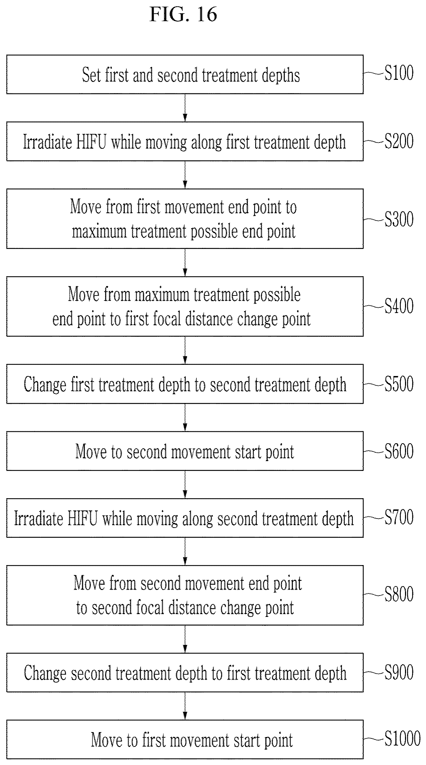

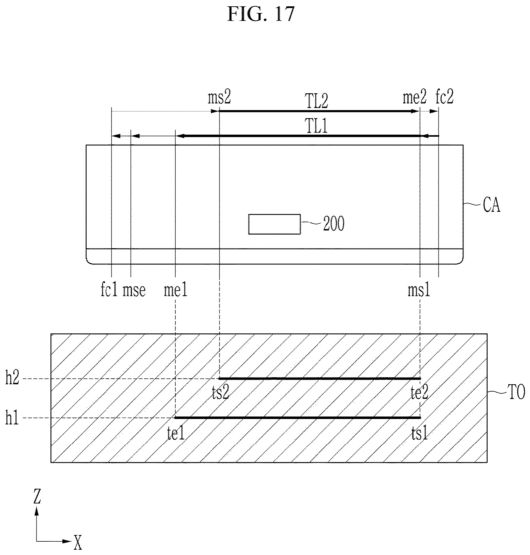

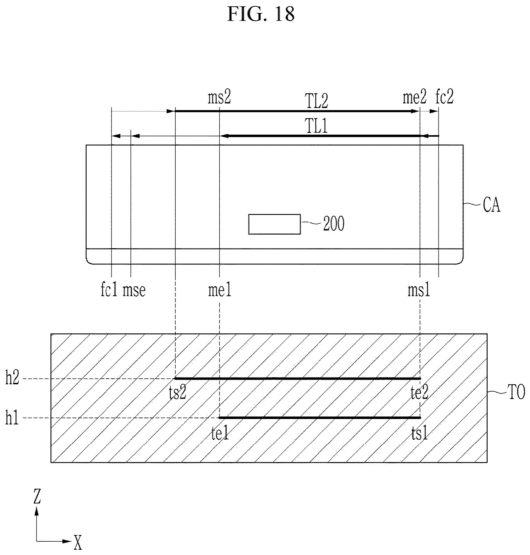

FIG. 16 is a schematic flowchart of a control method of a cartridge for an ultrasound apparatus according to an embodiment, FIG. 17 is provided for description of an embodiment of the control method of the cartridge for the ultrasound apparatus of FIG. 16, and FIG. 18 is provided for description of another embodiment of the control method of the cartridge for the ultrasound apparatus of FIG. 16.

As shown in FIG. 16 and FIG. 17, a control method of a cartridge for an ultrasound apparatus according to an embodiment sets a first treatment length TL1 and a second treatment length TL2 (S100).

Here, the first treatment length TL1 may be a distance between a first treatment start point ts1 where a high-intensity focused ultrasound (HIFU) is irradiated at a first treatment depth h1, and which is the starting point of the treatment, and a first treatment end point te1, which is the point where the treatment ends, and the second treatment length TL2 may be a distance between a second treatment start point ts2, which is the starting point where high-intensity focused ultrasound (HIFU) is irradiated at a second treatment depth h2, and a second treatment end point te2, which is the ending point of the treatment.

The first treatment length TL1 and the second treatment length TL2 may be different from each other. In the embodiment shown in FIG. 17, the first treatment length TL1 is illustrated to be longer than the second treatment length TL2, but this is not restrictive, and as shown in the embodiment of FIG. 18, the first treatment length TL1 may be shorter than the second treatment length TL2.

Next, the first moving member 330 moves the transducer 200 from a first movement start point ms1 to a first movement end point me1 and irradiates a treatment target TO with the HIFU for the first treatment length TL1 (S200). Thus, the treatment may be performed in the treatment area corresponding to the first treatment length TL1. Here, since the transducer 200 irradiates the HIFU at the first treatment depth h1 from the first movement start point ms1 to the first movement end point me1, the first movement start point ms1 may correspond to the first treatment start point ts1, and the first movement end point me1 may correspond to the first treatment end point te1.

In this case, the first treatment end point te1 may be changed by adjusting the first movement end point me1 of the transducer 200. As described, by adjusting the first movement end point me1 of transducer 200, the first treatment length TL1 can be adjusted, thereby easily controlling the treatment area.

Next, using the first moving member 330, the transducer 200 is moved from the first movement end point me1 to a maximum treatment possible end point mse (S300). From the first movement end point me1 to the maximum treatment possible end point mse, only the transducer 200 is moved without irradiating the HIFU. The maximum treatment possible end point mse may be the maximum treatment possible point to which the transducer 200 can move and perform a treatment. When the transducer 200 is moved directly to a first focal distance change point fc1, the transducer 200 may collide with the cartridge main body 100, causing an impact to the transducer 200. To prevent this, the transducer 200 first moves to the maximum treatment possible end point mse and then reduces the speed.

Next, using the first moving member 330, the transducer 200 is moved from the maximum treatment possible end point mse to the first focal distance change point fc1 (S400). From the maximum treatment possible end point mse to the first focal distance change point fc1, only the transducer 200 is moved without irradiating the HIFU.

Next, the first treatment depth h1 is changed to the second treatment depth h2 by changing the focal distance of the transducer 200 using the second moving portion 340 (S500). In this case, the second moving portion 340 may change the focal distance of the transducer 200 without moving the transducer 200 to the left or to the right along the first direction X.

Next, using the first moving member 330, the transducer 200 is moved from the first focal distance change point fc1 to the second movement start point ms2 (S600). In this case, only the transducer 200 is moved without irradiating the HIFU.

Next, using the first moving member 330, the transducer 200 is moved from the second movement start point ms2 to the second movement end point me2, and the HIFU is irradiated to the treatment target TO for the second treatment length TL2 (S700). Thus, the treatment may be performed in the treatment area corresponding to the second treatment length TL2. Here, since the transducer 200 irradiates the HIFU at second first treatment depth h2 from the second movement start point ms2 to the second movement end point me2, the second movement start point ms2 may correspond to the second treatment start point ts2, and the second movement end point me2 may correspond to the second treatment end point te2. In this case, the second movement end point me2 may be the same as the first movement end point ms1.

Here, the second treatment start point ts2 may be changed by adjusting the second movement start point ms2 of the transducer 200. As described, the second treatment length TL2 may be adjusted by adjusting the second movement start point ms2 of the transducer 200, thereby easily controlling the treatment area.

Next, the transducer 200 is moved from the second movement end point me2 to the second focal distance change point fc2 using the first moving member 330 (S800). From the second movement end point me2 to the second focal distance change point fc2, only the transducer 200 is moved without irradiating the HIFU.

Next, the second treatment depth h2 is changed to the first treatment depth h1 transducer 200 by changing the focal distance (S900). In this case, the second moving portion 340 may change the focal distance of the transducer 200 without moving the transducer 200 to the left or to the right along the first direction X.

Next, the transducer 200 is moved from the second focal distance change point fc2 to the first movement start point ms1 using the first moving member 330 (S1000).

As described, the first moving member 330 may reciprocally move the transducer 200 left or right along the first direction X between the first focal distance change point fc1 and the second focal distance change point fc2.

Next, the procedure is performed by repeating the above-described steps.

Meanwhile, in the embodiment illustrated in FIG. 16 to FIG. 18, the first treatment end point te1 and the second treatment start point ts2 are set differently, but the first treatment end point te1 and the second treatment start point ts2 may be set to be the same.

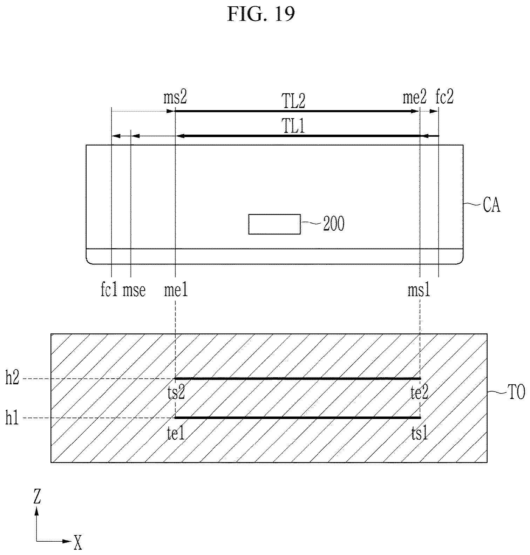

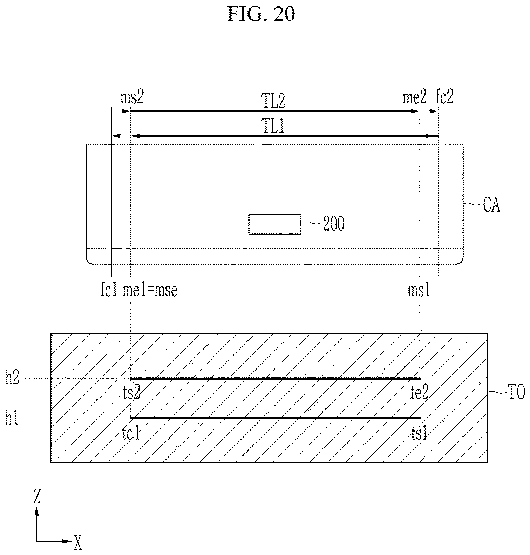

FIG. 19 and FIG. 20 are provided for description of still another embodiment of the cartridge for the ultrasound apparatus of FIG. 16.

As shown in FIG. 19, the first treatment end point te1 corresponding to the first movement end point me1 and the second treatment start point ts2 corresponding to the second movement start point ms2 may be set to be the same.

In this case, the first treatment end point te1 and the second treatment start point ts2 may be set to be the same as the first movement end point me1.

Therefore, the first treatment length TL1 and the second treatment length TL2 may be equal to each other. In addition, as the first treatment end point te1 and the second treatment start point ts2 are close to the maximum treatment possible end point mse, the treatment area can be increased.

Meanwhile, as shown in FIG. 20, the transducer 200 is moved such that the first movement end point me1 is equal to the maximum treatment possible end point mse, and therefore the first treatment end point te1 and the second treatment start point ts2 can be set equal to the maximum treatment possible end point mse. In this case, treatment efficiency can be maximized by maximizing the treatment area.

Although the present disclosure has been described preferably through embodiments as described above, those skilled in the art will readily understand that the present invention is not limited thereto and that various modifications and variations are possible as long as they do not depart from the scope of the patent claims described below.

## Claims

1. A cartridge for an ultrasound apparatus, comprising:
a cartridge main body;
a transducer that is provided in the cartridge main body and generates a high-intensity focused ultrasound; and
a moving device that is provided in the cartridge main body and moves the transducer,
wherein the moving device comprises:
a moving main body moving along a first direction;
a moving block that is connected to the moving main body and moving together with the transducer by being engaged with the transducer;
a first moving member that moves the moving block along the first direction and moves a focus of the high-intensity focused ultrasound along the first direction; and
a second moving portion that moves the moving block along a direction inclined with respect to and moves the focus of the high-intensity focused ultrasound along a second direction that is perpendicular to the first direction.

2. The cartridge for the ultrasound apparatus of claim 1, wherein:
the first moving member comprises:
a moving connection portion that connects a driver of a hand piece main body providing driving torque and the moving block; and
a pair of guide rods that are connected with the moving connection portion and guide the moving block to move along the first direction.

3. The cartridge for the ultrasound apparatus of claim 2, wherein:
the moving connection portion comprises:
a connection main body that reciprocally moves along the first direction;
a first connection portion that extends from an upper end of the connection main body and is connected with the driver;
a second connection portion that is connected with the pair of guide rods at a central portion of the connection main body; and
a third connection portion that is engaged with the moving block at a lower end of the connection main body.

4. The cartridge for the ultrasound apparatus of claim 3, wherein:
the second moving portion comprises:
a moving pin that connects the moving main body and the moving block and is movable;
a fixing pin that is installed in the moving main body in parallel at a distance from the moving pin; and
a link that connects the moving pin and the fixing pin to each other, and
the moving pin is reciprocally movable along an inclined hole formed along the inclined direction on a side wall of the moving main body.

5. The cartridge for the ultrasound apparatus of claim 4, wherein:
one end of the link includes a moving hole to which the moving pin is coupled,
the other end of the link includes a rotation hole to which the fixing pin is coupled, and
a size of the moving hole is larger than a diameter of the moving pin.

6. The cartridge for the ultrasound apparatus of claim 4, wherein:
the moving device further comprises a stationary member that is provided in an inner wall of the cartridge main body and stops the movement of the moving main body to the first direction, and
the stationary member comprises a first stationary member and a second stationary member that are opposite to each other in the first direction.

7. The cartridge for the ultrasound apparatus of claim 6, wherein:
the moving main body comprises:
a frame including the inclined hole; and
a first protruding portion extending in the first direction from the frame and is capable of being in contact with the stationary member.

8. The cartridge for the ultrasound apparatus of claim 7, wherein:
the moving main body further comprises a second protruding portion extending in the second direction from an upper end of the frame and to which the pair of guide rods are inserted,
the second protruding portion includes a second protruding portion on one side and a second protruding portion on the other side positioned spaced apart from each other by a distance, and
a length of the second connection portion is shorter than the distance.

9. The cartridge for the ultrasound apparatus of claim 3, wherein:
the second moving portion comprises a moving pin that connects the moving main body and the moving block and is movable, and
the moving pin is reciprocally movable along a two-stage guide hole formed on a side wall of the moving main body.

10. The cartridge for the ultrasound apparatus of claim 9, wherein:
the moving main body comprises:
a frame including the two-stage guide hole; and
a second protruding portion extending in the second direction from an upper end of the frame and to which the pair of guide rods are inserted.

11. The cartridge for the ultrasound apparatus of claim 10, wherein:
the second protruding portion includes a second protruding portion on one side and a second protruding portion on the other side positioned spaced apart from each other by a distance, and
a length of the second connection portion is shorter than the distance.

12. The cartridge for the ultrasound apparatus of claim 9, wherein:
the two-stage guide hole comprises
a first horizontal guide hole and a second horizontal guide hole that are disposed spaced apart from each other along the first direction and are formed at different heights along the second direction, and
an inclined connection hole formed along the slope direction and connecting the first horizontal guide hole and the second horizontal guide hole.

13. A cartridge for an ultrasound apparatus comprising:
a cartridge main body;
a transducer that is provided in the cartridge main body and generates a high-intensity focused ultrasound; and
a moving device that is provided in the cartridge main body and moves the transducer,
wherein the moving device comprises:
a fixed main body that is fixed within the cartridge main body and includes a moving guide groove on the inside;
a moving block that is connected to the moving main body and moving together with the transducer by being engaged with the transducer;
a first moving member that moves the moving block along the first direction and moves a focus of the high-intensity focused ultrasound along the first direction; and
a second moving portion that moves the moving block along a direction inclined with respect to and moves the focus of the high-intensity focused ultrasound along a second direction that is perpendicular to the first direction.

14. The cartridge for the ultrasound apparatus of claim 13, wherein:
the first moving member comprises:
a moving connection portion that connects a driver of a hand piece main body providing driving torque and the moving block; and
a pair of guide rods that are provided in the fixed main body and connected to the moving connection portion to guide the moving block to move along the first direction.

15. The cartridge for the ultrasound apparatus of claim 14, wherein:
the moving connection portion comprises:
a connection main body that reciprocally moves along the first direction;
a first connection portion that extends from an upper end of the connection main body and is connected with the driver;
a second connection portion that is connected with the pair of guide rods at a central portion of the connection main body; and
a third connection portion that is engaged with the moving block at a lower end of the connection main body.

16. The cartridge for the ultrasound apparatus of claim 15, wherein:
the moving guide groove comprises:
a first horizontal guide groove and a second horizontal guide groove that are formed at different heights and face each other; and
a first inclined guide groove and a second inclined guide groove that are formed along an inclination direction, facing each other and connecting the first horizontal guide groove and the second horizontal guide groove.

17. The cartridge for the ultrasound apparatus of claim 16, wherein:
the second moving portion comprises:
a moving link that is connected with the third connection portion and moves along the movement guide groove by being inserted into the moving guide groove;
a connection pin that connects the moving link and the third connection portion; and
a middle pin connecting the moving link and the moving block.

18. A control method of a cartridge for an ultrasound apparatus that adjusts a treatment depth of high-intensity focused ultrasound generated from a transducer and irradiated to a treatment target, comprising:
setting a first treatment length, which is a distance between a first treatment start point where the high-intensity focused ultrasound is irradiated at a first treatment depth and a first treatment end point, and a second treatment length, which is a distance between a second treatment start point where the high-intensity focused ultrasound is irradiated at a second treatment depth that is different from the first treatment depth and a second treatment end point;
moving the transducer from a first movement start point to a first movement end point and irradiating the treatment target with the high-intensity focused ultrasound for the first treatment length;
changing the first treatment depth to the second treatment depth by changing a focal distance of the transducer;
moving the transducer to a second movement start point; and
irradiating the treatment target with the high-intensity focused ultrasound at the second treatment length while moving the transducer from the second movement start point to a second movement end point,
wherein the first treatment end point corresponding to the first movement end point is changeable by adjusting the first movement end point of the transducer.

19. The control method of the cartridge for the ultrasound apparatus of claim 18, wherein:
the first movement start point corresponds to the first treatment start point,
the second movement start point corresponds to the second treatment start point,
the second movement end point corresponds to the second treatment end point, and
the second treatment start point is changeable by adjusting the second movement start of the transducer.

20. The control method of the cartridge for the ultrasound apparatus of claim 18, further comprising:
moving the transducer from the first movement end point to a maximum treatment possible end point; and
moving the transducer to a first focal distance change point from the maximum treatment possible end point,
wherein the maximum treatment possible end point is a maximum treatment possible point to which the transducer performs treatment while moving.

21. The control method of the cartridge for the ultrasound apparatus of claim 20, further comprising:
moving the transducer to a second focal distance change point from the second movement end point;
changing the second treatment depth to the first treatment depth by changing the focal distance of the transducer; and
moving the transducer to the first movement start point.

22. The control method of the cartridge for the ultrasound apparatus of claim 21, wherein:
the second movement end point is the same as the first movement start point.

23. The control method of the cartridge for the ultrasound apparatus of claim 21, wherein:
the first treatment end point and the second treatment start point are set to be different from each other.

24. The control method of the cartridge for the ultrasound apparatus of claim 21, wherein:
the first treatment end point and the second treatment start point are set to be the same.

25. The control method of the cartridge for the ultrasound apparatus of claim 24, wherein:
the first treatment end point and the second treatment start point are set to be the same as the first movement end point.

26. The control method of the cartridge for the ultrasound apparatus of claim 24, wherein:
the first treatment end point and the second treatment start point are set to be the same as the maximum treatment possible end point.

27. The control method of the cartridge for the ultrasound apparatus of claim 21, wherein:
a moving device that moves the transducer comprises:
a moving block that is engaged with the transducer and moves together with the transducer; and
a first moving member that moves a focus of the high-intensity focused ultrasound along a first direction while moving the moving block along the first direction,
wherein the first moving member reciprocally moves the transducer between the first focal distance change point and the second focal distance change point.

28. The control method of the cartridge for the ultrasound apparatus of claim 27, wherein:
the moving device further comprises a second moving point that moves a focus of the high-intensity focused ultrasound in a second direction that is perpendicular to the first direction while moving the moving block along an inclined direction that is inclined with respect to the first direction, and
the second moving portion changes a focal distance of the transducer.
